# EUROPÄISCHE PATENTSCHRIFT

(11) **EP 1 959 871 B1**
(45) Veröffentlichungstag und Bekanntmachung des Hinweises auf die Patenterteilung: **20.03.2013**
(21) Anmeldenummer: 06819030.5
(22) Anmeldetag: 13.12.2006
(51) Int. Cl.: A61F 2/44, A61F 2/30

(54) **FACETTENGELENKPROTHESE**
FACET JOINT PROSTHESIS
PROTHESE D'ARTICULATION A FACETTES

(30) Priorität: 13.12.2005 DE 202005019487 U
(43) Veröffentlichungstag der Anmeldung: 27.08.2008
(73) Patentinhaber: FACET-LINK Inc., Rockaway NJ 07866 (US)
(72) Erfinder: LINK, Helmut, D., 22397 Hamburg (DE)
(74) Vertreter: Glawe, Delfs, Moll
(86) Internationale Anmeldenummer: PCT/EP2006/012007
(87) Internationale Veröffentlichungsnummer: WO 2007/068469

(56) Entgegenhaltungen:
- WO-A-2006/065774
- US-A1- 2003 004 572
- US-A1- 2005 119 748
- US-A1- 2005 177 240

## Beschreibung

Die Erfindung betrifft eine Prothese für Facettengelenke der Wirbelsäule mit einem Lagerstück und einem Haltestück.

Rückenbeschwerden sind häufig auf pathologische Veränderungen im Bereich der Wirbelsäule zurückzuführen. Einer der Hauptursachen liegt in der Interaktion zwischen zwei benachbarten Wirbeln und ihrer Verbindung. Dies betrifft insbesondere Bandscheiben und Facettengelenke. Aufgrund von Krankheit oder Überbeanspruchung, sei es durch Verletzung oder Verschleiß, kann die Verbindung zwischen Wirbeln beschädigt sein. Dies kann zu Bewegungseinschränkungen, Schmerzen bis hin zu einem Verlust der Mobilität führen. In der Vergangenheit fokussierte sich die Behandlung auf eine Therapie der Bandscheiben. So sind verschiedene künstliche Gelenke bekannt geworden, die anstelle einer defekten natürlichen Bandscheibe implantiert werden. Damit könnten indirekt auch Defekte an den Facettengelenken ausgeglichen bzw. deren Folgen gemindert werden. Es hat sich aber gezeigt, dass dies nicht ausreichend ist, sondern dass in vielen Fällen auch eine gezielte Therapie der Facettengelenke erforderlich ist.

Zur Behandlung im Bereich der Facettengelenke sind verschiedene Ansätze versucht worden. Im einfachsten Fall beschränkt sich die Therapie auf eine Verminderung der durch das Facettengelenk verursachten Schmerzen, insbesondere durch Injektion von schmerzlindernden Mitteln im Bereich des betroffenen Facettengelenks. Eine weitere besteht darin, das Facettengelenk zu entfernen. Damit ist zwar eine der Ursachen der Schmerzen beseitigt, jedoch um den Preis einer signifikanten Veränderung der Biomechanik der Wirbelsäule. Fehlende Facettengelenke reduzieren die Stabilität der Wirbelsäule im erheblichen Maße und führen damit zu einer stärkeren Beanspruchung der übrigen Bereiche der Wirbelsäule, was nicht selten zu Defekten im Bereich benachbarter Verbindung zwischen Wirbeln führt. Ähnliches gilt für den umgekehrten Weg, das Facettengelenk durch eine feste Verbindung zu immobilisieren. Auch hierbei wird die Biomechanik in negativer Weise beeinflusst. Die Mobilität des Patienten verschlechtert sich, außerdem kommt es zu einer höheren Beanspruchung in dem Bereich der benachbarten Wirbel.

Weiter ist es bekannt geworden, die Facettengelenke im Bereich ihrer Gelenkfläche mit einer schützenden Kappe zu versehen. Um Raum für die Kappe zu schaffen, muss in beträchtlichem Umfang Material von der natürlichen Lagerfläche abgetragen werden. Dieses Entfernen von Gewebe führt häufig zu einer Unterbrechung der natürlichen Versorgung von umliegendem gesunden Geweben, mit der Folge nekrotischer Veränderungen.

Um diese Nachteile dieser traditionellen Therapieverfahren zu vermeiden, sind verschiedene Prothesen für Facettengelenke entwickelt worden.

Eine aufwendige Bandscheiben-Prothese mit daran ansteckbaren zusätzlichen Facettengelenk-Prothesen ist aus der WO-A-2004/098465 bekannt. Die Prothese eignet sich damit zur gleichzeitigen Therapie der Bandscheibe und der Facettengelenke. Durch diese Kombination ist die Prothese in ihrem Aufbau kompliziert und weist verhältnismäßig große Abmessungen auf. Dies bedingt eine aufwendige Operation mit groß dimensioniertem Zugang. Eine Therapie der Facettengelenke im Bereich der Halswirbelsäule ist mit dieser Prothese schwierig.

Eine kompaktere Prothese für ein Facettengelenk ist aus der WO-A-2005/037149 bekannt. Sie weist ein elastisches Element mit einer scheibenartigen Verdickung auf, die zwischen den Lageroberflächen des Facettengelenks platziert ist. Die Befestigung der Prothese erfolgt durch Befestigungselemente, die in Durchgangslöchern in den entsprechenden Gelenktortsätzen eingebracht sind. Da diese Durchgangslöcher miteinander fluchten müssen, ist ihre Schaffung recht schwierig und fordert einen ungehinderten Zugang. Weiter schafft die Prothese eine flexible, aber ursprünglich nicht vorhandene mechanische Zugverbindung des Facettengelenks. Die Biomechanik wird insoweit verändert.

Eine weitere spezielle Facettengelenk-Prothese ist aus der WO-A-2004/103227 bekannt. Sie besteht aus zwei Komponenten, die jeweils ein Lagerstück und ein Haltestück zur Verankerung an den benachbarten Wirbeln aufweisen. Eine obere Komponente ist mittels einer Schraube in der Lamina des Wirbels verankert. Die Schraube weist einen verhältnismäßig langen Schaft auf, der zu ungünstigen Hebelverhältnissen und damit zu einer Belastung der Lamina führt. Um ein Knicken der Schraube zu vermeiden, muss sie verhältnismäßig groß dimensioniert sein. Die Prothese weist viele herausstehende Teile vor, was die Gefahr von Irritationen umliegenden Gewebes mit sich bringt. Außerdem bringt die Befestigung der oberen Lagerkomponente an der Lamina statt an dem entsprechenden Gelenkfortsatz eine Veränderung der Biomechanik mit sich. Eine ähnliche Facettengelenk-Prothese mit einer translaminaren Befestigungsschraube ist aus der US-A-2005/0049705 bekannt. Die Befestigungsschraube liegt mit ihrem Kopf auf der Lamina auf und läuft durch einen im Wirbel geschaffenen Kanal zu der unteren Facettengelenkfläche, wo ein Lagerstück angeordnet ist und mittels der Befestigungsschraube fixiert wird. Das Lagerstück wirkt zusammen mit einem an dem benachbarten Wirbel angeordneten Gegenstück des Facettengelenks, welches mittels eines einfachen Doms an dem Wirbel befestigt ist. Ein Nachteil dieser Prothese ist, dass die Schaffung des translaminaren Kanals recht aufwendig ist, und dass eine passgenaue Fügung von Lagerstück und Schraube schwierig ist, da sie von dem jeweiligen Winkel der Facettengelenkfläche abhängt. Besonders im zervikalen Bereich der Wirbelsäule ergeben sich damit erhebliche Schwierigkeiten in der Handhabung ergeben.

Aus der US-A-2004/0049272 ist eine Facettengelenk-Prothese bekannt, die ein Lagerstück mit einem starr daran angeordneten und in den Wirbel eintauchenden Haltestab umfasst. Das Lagerstück weist eine tassenartige Wölbung auf und umfasst eine Druckplatte zur Auflage auf dem Wirbel und eine Lagerschale, die mit einem filzartigen Gegenstück an der entsprechenden Gegenfläche des Facettengelenks zusammenwirkt. Der Haltestab kann fest oder abnehmbar mit dem Lagerstück verbunden sein. Aber auch diese Prothese ist in der Handhabung gerade bei beengten räumlichen Verhältnissen, wie sie im zervikalen Bereich vorherrschen, diffizil, wenn Haltestab und Gelenkstück in situ zusammengesetzt werden müssen. Sind sie vormontiert, so stellt sich die Schwierigkeit des Einbringens unter den beengten räumlichen Verhältnissen. Zur sicheren Fixierung muss der Haltestab weit in den Bereich des Wirbelkörpers hineinragen, was dort zu Irritationen führen kann.

Aus der US-A-0 177 240 ist eine Facettengelenk-Prothese bekannt, die ein Lagerstück mit einem daran anschraubbaren Führungsstab umfasst. Das Lagerstück ist an seiner Unterseite als Druckplatte zur Auflage auf dem Wirbel und an seiner gegenüberliegenden Seite als Lagerfläche zum Zusammenwirken mit einer Gegengelenkfläche ausgebildet. Der Führungsstab weist an seinem gegenüberliegenden Ende ein Gegenelement auf, das mit einer Schraubendreheraufnahme versehen sein kann. Die Befestigung des Führungsstabs an dem Lagerstück geschieht mittels einer Schraubverbindung. Unter den beengten Verhältnissen, gerade im Bereich der Halswirbelsäule, kann die Schaffung einer entsprechenden Durchgangsbohrung an exakter Position sowie das Verbinden des Lagerstücks mit dem Führungsstab beträchtliche Schwierigkeiten aufwerfen.

Der Erfindung liegt die Aufgabe zugrunde, eine verbesserte Prothese für Facettengelenke zu schaffen, die weniger invasiv ist und einer dem natürlichen Facettengelenk näher kommende Biomechanik aufweist.

Die erfindungsgemäße Lösung liegt in den Merkmalen der unabhängigen Ansprüche. Vorteilhafte Weiterbildungen sind Gegenstand der abhängigen Ansprüche.

Bei einer Prothese für Facettengelenke der Wirbelsäule mit einem Lagerstück und einem Führungsstab als Haltestück, dessen Lagerstück eine Druckplatte zur Auflage auf dem Wirbel und gegenüberliegend eine Lagerschale umfasst, die eine Gelenkfläche zum Zusammenwirken mit einer Gelenkfläche eines benachbarten Wirbels aufweist, ist erfindungsgemäß zur Befestigung des Führungsstabs an dem Lagerstück ein Schwenkgelenk vorgesehen. Am anderen Ende des Führungsstabs ist ein zweites Lagerstück mit Druckplatte und Lagereinsatz angeordnet.

Kern der Erfindung ist der Gedanke, eine Prothese zu schaffen, bei der einerseits die Funktion des Haltens der Prothese an ihrem vorgesehenen Ort entkoppelt ist von der Funktion des Aufnehmens der Lagerkräfte, und die andererseits an verschiedene anatomische Verhältnisses anpassbar und damit leicht montierbar ist. Die Gelenkflächen der Facettengelenke sind in unterschiedlicher Neigung an den Wirbeln ausgebildet. Brustwirbel haben eine andere Neigung als beispielsweise Halswirbel. Um diese Unterschiede und außerdem individuelle Unterschiede zwischen verschiedenen Patienten leicht ausgleichen zu können, ist der Führungsstab über Schwenkgelenke an den Lagerstücken befestigt. Dies ermöglicht eine Anpassung des Winkels an die jeweiligen anatomischen Gegebenheiten. Die Erfindung benötigt keinen exakt auf die Lagerflächen in einem bestimmten Winkel ausgerichteten Kanal, in den eine Befestigungsschraube eingebracht werden und mit dem Lagerstück verbunden werden muss, sondern der Erfindung genügt vielmehr ein einfach zu schaffender Schlitz, dessen Winkelorientierung zu der Lagerfläche in weiteren Grenzen variieren kann. Sie erleichtert das Einsetzen der erfindungsgemäßen Prothese in der richtigen Stellung und ermöglicht eine operationstechnisch günstige Ausarbeitung des

Schlitzes an dem Wirbel, nämlich am Gelenkfortsatz in Richtung zwischen den oberen und unteren Gelenkflächen. Die erfindungsgemäße Facettengelenkprothese braucht lediglich in diesen Schlitz eingebracht zu werden, ohne dass eine komplizierte Montage oder Verbindung der verschiedenen Elemente zu erfolgen braucht, insbesondere muss nicht wie im Stand der Technik der Führungsstab mittels einer Schraubverbindung mit dem Lagerstück verbunden werden. Die Erfindung eignet sich damit besonders zur Implantation an schwer zugänglichen und räumlich beengten Stellen, wie insbesondere im Bereich der Halswirbelsäule. Aufgrund dieser Einfachheit der Montage kann die erfindungsgemäße Prothese klein ausgeführt werden. Dies ermöglicht es, mit einem kleinen Führungsstab und damit einem kleinen Schlitz auszukommen. Die dazu erforderlichen Bearbeitungen des Wirbels sind minimal. Der Schlitz kann von der Seite in den Wirbel eingebracht sein, vorzugsweise von dorso-lateral. Damit ermöglicht diese Prothese einen besonders günstigen Zugangsweg. Die erfindungsgemäße Prothese erlaubt insgesamt einen weitgehenden Erhalt der natürlichen Substanz. Damit eignet sich die erfindungsgemäße Prothese für eine Operation, die nur wenig invasiv ist. Die erfindungsgemäße Prothese ermöglicht sogar- bei entsprechend gewählter Wölbung der Lagerfläche - einen einseitigen Teilersatz, also das Zusammenwirken mit der natürlichen Gelenkfläche des benachbarten Wirbels. Außerdem bleibt mit der erfindungsgemäßen Prothese das kinematische Zentrum des Gelenks an seinem ursprünglichen Ort. Damit verknüpft die erfindungsgemäße Prothese eine günstige Biomechanik mit den Vorteilen einer vergleichsweise leichten Implantierbarkeit, die auch nur wenig invasiv ist. Weiter wird durch die Anordnung des zweiten Lagerstückes am anderen Ende des Führungsstabs eine Prothese gebildet, mit der Facettengelenke in aneinander liegenden Etagen therapierbar sind. Dabei ist nur ein Schlitz erforderlich, in den der Führungsstab der Prothese einzusetzen ist. Dank der an beiden Enden des Führungsstabs angeordneten Druckplatten wird eine zusätzliche Befestigungssicherheit erreicht.

Zur feineren Anpassung an individuell unterschiedliche Anatomien kann es aber von Vorteil sein, wenn das Schwenkgelenk in einer zweiten Richtung quer zur Längsachse des Führungsstabs beweglich ist. Damit ist eine Beweglichkeit in der Frontalebene erreicht. Dazu kann das Schwenkgelenk als Kugelgelenk ausgebildet sein, oder vorzugsweise als Kardangelenk.

Der Führungsstab ist zweckmäßigerweise in seiner Mittellage schrägwinklig zur Druckplatte an dem Lagerstück angeordnet. Damit ist eine anatomisch günstige Form erreicht. In der Regel genügt es, wenn das Schwenkgelenk in einer Richtung quer zur Längsachse des Führungsstabs beweglich ist. Damit ist eine Beweglichkeit in der Sagittalebene erreicht.

Im einfachsten Fall weist der Führungsstab einen runden Querschnitt auf. Zweckmäßig ist es aber, wenn er einen unrunden, vorzugsweise rechteckigen oder ovalen Querschnitt aufweist. Die Abmessungen sind so gewählt, dass die Breite des Führungsstabs je nach Orientierung kleiner oder größer als die Weite des Schlitzes ist. Dies ermöglicht es, den Führungsstab in seiner schmalen Orientierung in den Schlitz einzubringen, und an seinen vorgesehenen Ort so weit um seine Längsachse zu verdrehen, bis er in dem Schlitz verklemmt ist. Dadurch ist er gegenüber einem unbeabsichtigten Herausrutschen geschützt. Bei einem rechteckigen Querschnitt kann dabei dank der Kanten ein Einrasteffekt erreicht werden, wodurch sich eine zusätzliche Sicherung ergibt. Unbedingt nötig ist der unrunde Querschnitt aber nicht, da dank der erfindungsgemäß möglichen kleinen Dimensionierung des Schlitzes in der Regel mit einem schnellen Zuwachsen gerechnet werden kann. Um die erfindungsgemäße Prothese weiter vor unerwünschter Dislokation zu schützen, kann zweckmäßigerweise entlang des Führungsstabs eine radial hervorstehende Zackung ausgebildet sein. Sie sichert den Führungsstab zusätzlich gegenüber einem unbeabsichtigten seitlichen Herausrutschen. Zu diesem Zweck kann auch eine Lasche an dem Führungsstab angeordnet sein, die zur Befestigung am Wirbel dient, beispielsweise mittels einer Verschraubung.

Um das Lagerstück zusätzlich an dem Wirbel zu sichern, ist zweckmäßigerweise an der Druckplatte eine zum Eingreifen in den Wirbel ausgebildete Verzahnung vorgesehen. Damit drückt sich das Lagerstück unter Einwirkung der Lagerkräfte selbsttätig in den Wirbel ein. Dadurch ist es gegenüber Verschiebungen oder Verdrehungen zusätzlich gesichert. Die Verzahnung kann aus einzelnen Zähnen bestehen, oder es kann eine Zahnreihe vorgesehen sein, beispielsweise in kreisförmiger Anordnung. Um die Verbindung mit dem Wirbel weiter zu verbessern, kann außerdem noch eine Knochenwachstum fördernde Beschichtung an der Druckplatte vorgesehen sein. Ein Beispiel für eine solche Beschichtung ist Hydroxylapatit. Dies begünstigt das Einwachsen der Prothese an den Wirbeln.

Die gegenüberliegend der Druckplatte an dem Lagerstück ausgebildete Gelenkschale weist vorzugsweise eine konvexe oder konkave Wölbung auf. Welche Wölbung angemessen ist, hängt von der anatomischen Form des entsprechenden Wirbels ab. Zweckmäßigerweise wird sie so gewählt, dass sie auf die Form der an dem benachbarten Wirbel angeordneten Gelenkfläche, mit der zusammen die Prothese das Facettengelenk bildet, abgestimmt ist.

Der Führungsstab ist vorzugsweise mehrteilig ausgebildet. Zweckmäßig ist eine teleskopartige Gestaltung des Schafts. Damit kann die Länge des Führungsstabs auf die Dicke des Wirbels, genauer gesagt auf die Länge des Schlitzes von einer Gelenkfläche des Wirbels zu der anderen, abgestimmt werden. Das Teleskop kann durch konzentrisch ineinander laufende Elemente gebildet sein, oder durch eine Schraubverbindung. Um ein unerwünschtes Auseinanderbewegen zu verhindern, wodurch die Gefahr einer Lockerung der Lagerstücke von ihrem Sitz auf den Wirbeln hervorgerufen würde, sind zweckmäßigerweise Verriegelungsmittel für die Schaftteile des Führungsstabs vorgesehen. Dazu können gesonderte Rastelemente vorgesehen sein. Es kann aber auch eine solche Gewindesteigung für die Schraubverbindung gewählt werden, die eine Selbsthemmung bewirkt. Die Schraubverbindung bietet den Vorzug, dass durch einfaches Verdrehen eines der Lagerstücke die Länge des Führungsstabs verändert werden kann, um so einen festen Sitz der Lagerstücke zu erreichen. Zweckmäßigerweise ist dazu an wenigstens einem der Lagerstücke eine Aufnahme für einen Schraubschüssel vorgesehen.

Die Lagerstücke an den beiden Enden des Führungsstabs können verschieden gestaltet sein. Besonders zweckmäßig ist es, das Lagerstück an einem (superioren) Ende des Führungsstabs eine Kontur mit einer kreisartigen Umhüllenden und dem Lagerstück an dem anderen (inferioren) Ende eine Kontur mit einer rechteckigen Umhüllenden zu geben. Hierbei sind die Begriffe kreisartig und rechteckig nicht im streng mathematischen, sondern im verallgemeinerten Sinne zu verstehen. Insbesondere kann das Rechteck abgerundete Ecken aufweisen, so dass es eine ovalartige Form aufweist. Mit dieser Gestaltung kann eine bessere Annäherung an die Form der natürlichen Gelenkflächen der Facettengelenke erreicht werden.

Die erfindungsgemäße Prothese eignet sich insbesondere zur Therapie in mehreren aneinandergrenzenden Etagen von Wirbeln. Dazu ist vorzugsweise ein Prothesensatz vorgesehen, der gebildet ist aus mindesten einer Prothese mit zwei Lagerstücken und zwei Prothesen mit jeweils einem Lagerstück. Damit können Facettengelenke in zwei benachbarten Etagen therapiert werden. Es können durch zusätzliche Verwendung von Prothesen mit zwei Lagerstücken aber auch noch weitere Etagen hinzugefügt werden. In dieser Fähigkeit zur Therapie von Multietagen-Pathologien liegt ein besonderer Vorteil der Erfindung. Mit der erfindungsgemäßen Prothese bleibt die natürliche Biomechanik weitestgehend erhalten, so dass auch bei einer Verwendung über mehrere Etagen dank der guten biomechanischen Eigenschaften der erfindungsgemäßen Prothese weder eine Einschränkung der Mobilität, noch andererseits ein Verlust der Stabilität zu befürchten ist.

Die Erfindung wird nachfolgend unter Bezugnahme auf die beigefügte Zeichnung erläutert, in der vorteilhafte Ausführungsbeispiele dargestellt sind. Es zeigen:
- Fig. 1 a)-c): eine Seiten-, Vorder- und Rückansicht einer illustrativen Ausführungsform;
- Fig. 2: eine Querschnittsansicht längs einer Linie II-II aus Fig. 1;
- Fig. 3: eine vergrößerte Seitenansicht eines Bereichs der Halswirbelsäule mit implantierter Prothese gemäß einer illustrativen Ausführungsform;
- Fig. 4a)-b): eine Teillängsschnitt- und eine Querschnittansicht einer weiteren Variante der illustrativen Ausführungsform;
- Fig. 5a)-b): eine Vorder- und Rückansicht einer Ausführungsform der erfindungsgemäßen Prothese;
- Fig. 6: eine Teilansicht der Halswirbelsäule mit implantierter Halswirbelprothese gemäß der erfindungsgemäßen Ausführungsform;
- Fig. 7: eine Seitenansicht eines vorgesehenen Implantationsort einer Halswirbelsäule;
- Fig. 8: eine vergrößerte Darstellung eines Halswirbels mit Erläuterungen.

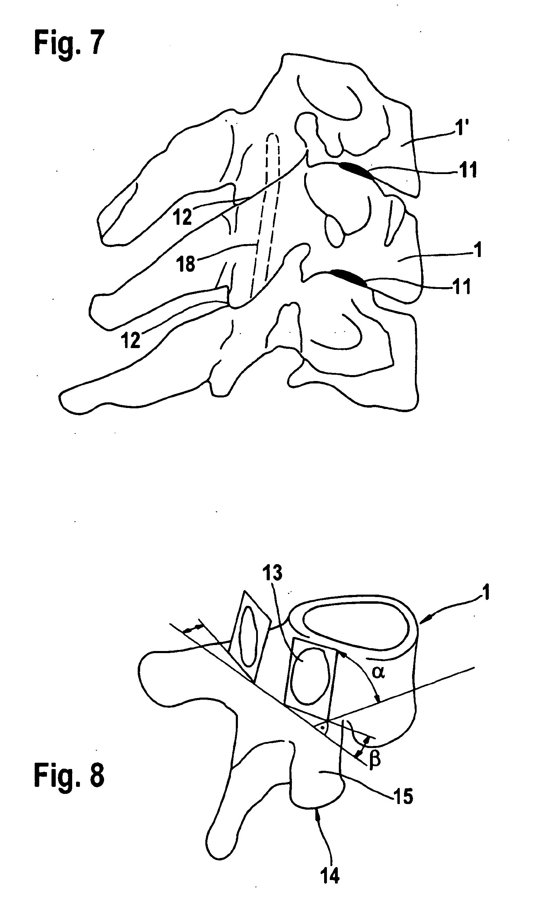

Zum leichteren Verständnis der Erfindung sei zuerst der vorgesehene Implantationsort erläutert. Die Prothese ist vorgesehen für Facettengelenke der Wirbelsäule. In Fig. 7 ist ein Ausschnitt der Wirbelsäule eines Menschen dargestellt, genauer gesagt, ein Ausschnitt aus der Halswirbelsäule. Man erkennt eine Mehrzahl (drei) von übereinander angeordneten Wirbeln. Die Wirbel umschließen in ihrem vorderen Bereich (in der Darstellung rechts) einen Markkanal, in dem auf- und absteigende Nervenbündel laufen. In ihrem hinteren Bereich (in der Darstellung links) weisen die Wirbel einen rückwärtigen Fortsatz (*processus spinosus*) auf. An den Seiten der Wirbel befindet sich jeweils ein Gelenkfortsatz (*processus articularis*) 15. Dieser weist jeweils zwei Gelenkflächen auf, eine obere Gelenkfläche (*facies articularis superior*) 13 im oberen Bereich und eine untere Gelenkfläche (*facies articularis inferior*) 14 im unteren Bereich. Die obere und unter Gelenkfläche 13, 14 zweier übereinander angeordneter Wirbel 1, 1' wirken miteinander zusammen. Sie bilden gemeinsam ein Facettengelenk 12. Übereinander angeordneten Wirbel 1, 1' sind jeweils über zwei Facettengelenke 12 und eine Bandscheibe 11 miteinander verbunden.

Die oberen Gelenkflächen 13 und ihre Anordnung an dem Wirbel 1 sind in Fig. 8 näher dargestellt. Die oberen Gelenkflächen 13 befinden sich an dem Gelenkfortsatz (*processus articularis*) 15. Sie sind mit einem steilen Winkel α von ca. 80° gegenüber der Horizontalen an der rückwärtigen Seite des Wirbels 1 angeordnet. Außerdem sind die oberen Gelenkflächen 13 noch um einen Winkel nach außen gegenüber der Querachse verdreht. Dieser Winkel ist in Fig. 8 als Winkel β dargestellt. Er beträgt beispielsweise 20°. Entsprechend ist die untere Gelenkfläche (*facies articularis inferior*) 14 ausgestaltet. Sie ist unten an dem Gelenkvorsprung 15 angeordnet. Die untere Gelenkfläche 14 eines Wirbels 1' ist zu der oberen Gelenkfläche 13 des darunter angeordneten Wirbels 1 komplementär ausgestaltet.

Die Erfindung sieht den Ersatz von durch Krankheit oder Verletzung defekt gewordenen Gelenkflächen 13, 14 vor. Eine zur Verdeutlichung dienende Ausführungsform für eine

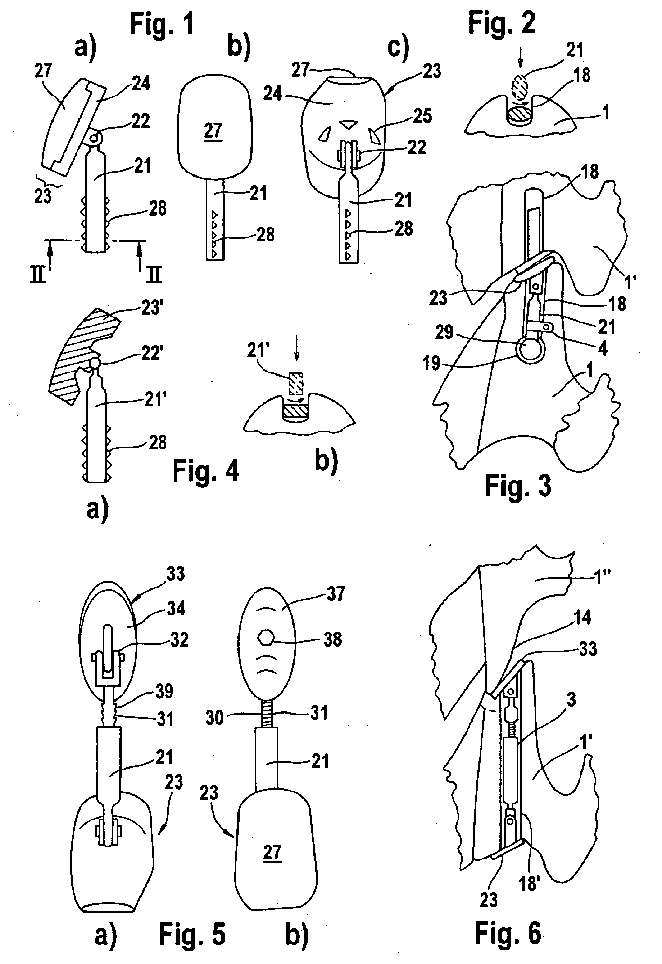

Prothese 2 ist in Fig. 1 a) - c) abgebildet. Die Prothese umfasst einen Führungsstab 21, der über ein als Bolzenlager ausgeführtes Schwenkgelenk 22 winkelbeweglich an einem Lagerstück 23 angeordnet ist. Das Lagerstück 23 umfasst an seiner dem Führungsstab 21 zugewandten Seite eine Druckplatte 24 und an seiner gegenüber liegenden Seite eine Lagerschale 27. Letztere kann aus einem gleitgünstigen Kunststoffmaterial (beispielsweise Polyethylen) bestehen. Optional kann vorgesehen sein, die Lagerschale 27 drehbeweglich an der Druckplatte 27 anzuordnen. Dazu weist die Druckplatte 24 an ihrer Oberseite eine topfartige Ausnehmung auf, in welche die Lagerschale 27 mit einer stumpfartig ausgebildeten Unterseite gelagert ist. Es ist aber ebenfalls möglich, die Lagerschale 27 aus einer biokompatiblen Metall-Legierung, beispielsweise CoCrNi auszuführen. Letzteres eröffnet die zweckmäßige Wirklichkeit, das Lagerstück 23 mit der Druckplatte 24 und der Lagerschale 27 einstückig auszuführen. Die zum Führungsstab 21 weisende Seite der Druckplatte 24 ist zur Auflage auf dem Wirbel 1, genauer gesagt auf der zur therapierenden Gelenkfläche 13, 14 ausgebildet. Zur besseren Verankerung der Druckplatte 24 an dem Wirbel 1 sind zweckmäßigerweise Hervorstehungen vorgesehen, die in der dargestellten Ausführungsform als eine ringförmig ausgeführte Verzahnung 25 ausgebildet ist. Im implantierten Zustand greift die Verzahnung 25 in die zu therapierende Gelenkfläche 13, 14 ein, und sie fixiert die Druckplatte 24 und damit das Lagerstück 23 der erfindungsgemäßen Prothese 2 an dem vorgesehenen Ort.

Der Führungsstab 21 dient dazu, die Position der Prothese 2 zu bestimmen. Es ist vorgesehen, an dem Gelenkfortsatz 15 des Wirbels 1 in den Bereich des zu therapierenden Facettengelenks 12 einen Schlitz 18 auszubilden. Dieser Schlitz 18 ist so orientiert, dass er in Richtung von der oberen Gelenkfläche 13 zu der unteren Gelenkfläche 14 durch den Gelenkfortsatz 15 läuft. Die Länge des Schlitzes 18 kann sich über die gesamte Höhe des Gelenkfortsatzes 15 erstrecken, oder wie in Fig. 3 abgebildet auf eine der zu therapierenden Gelenkfläche 13, 14 benachbarten Bereich. Der Querschnitt des Schlitzes 18 kann so bemessen sein, dass er zur Aufnahme des Führungsstabs 21 mitsamt dem Schwenkgelenk 22 ausreicht; bei einer versenkten Anordnung des Schwenkgelenks genügt es, wenn der Querschnitt zur Aufnahme des Führungsstabs 21 bemessen ist. Eine solche Variante mit einem Kugelgelenk 22' als Schwenkgelenk an einem einstückig ausgeführten Lagerstück 23' ist in Fig. 4a dargestellt. Die Prothese 2 kann durch einfaches Einbringen ihres Führungsstabs 21 in den Schlitz 18 am Gelenkfortsatz 15 an ihren Implantationsort bewegt werden. Dank des Schwenkgelenks 22 richtet sich das Lagerstück 23 dabei selbsttätig so aus, dass es einen der Neigung α und β entsprechenden Winkel einnimmt. Dadurch kommt die Druckplatte 24 in flächige Anlage auf die zu therapierenden Gelenkflächen 13, 14 des Gelenkfortsatzes 15. Die Druckplatte 24 überträgt auftretende Lagerkräfte in den Gelenkfortsatz 15. Der Führungsstab 21 dient im Wesentlichen nur zur Positionierung der Prothese 2, braucht aber im implantierten Zustand keine Lagerkräfte zu übertragen. Er kann damit klein dimensioniert sein. Dies ermöglicht es, die Abmessungen des Schlitzes 18 entsprechend klein zu wählen. Dies hat insbesondere den Vorteil, dass je geringer die Weite des Schlitzes 18 ist, desto eher und schneller ist mit einem Zuwachsen des Schlitzes 18 nach erfolgter Implantation zu rechnen. Damit wird die Prothese 2 auf eine besonders sichere und biokompatible Weise gegenüber einer unerwünschten Dislokation aus dem Schlitz 18 gehindert.

Um initial nach der erfolgten Implantation gleich eine möglichst gute Sicherheit gegenüber einem unerwünschten Herausrutschen der Prothese 2 zu haben, ist der Schaft des Führungsstabs 21 vorzugsweise nicht zylindrisch, sondern oval (Fig. 2) oder rechteckig (Fig. 4b) gestaltet. Dies ermöglicht es, bei der Implantation den Führungsstab 21 so zu orientieren, dass er mit seinem kleinen Querschnitt in den Schlitz 18 eingeschoben wird (siehe gestrichelte Linie in Fig. 2). Ist die Prothese 2 bis zu ihrem vorgesehenen Implantationsort in Schlitz 18 vorgeschoben, wird sie in ihrem Führungsstab 21 um 90° derart verdreht, dass sich der breite Querschnitt des Führungsstabs 21 über die Weite des Schlitzes 18 erstreckt. Zweckmäßigerweise ist die Weite des Schlitzes 18 dabei so gewählt, dass sie kleiner ist als die größte Weite des ovalförmigen Führungsstabs 21. Damit ergibt sich in dem verdrehten Zustand eine Verklemmung, die den Führungsstab 21 und damit die Prothese 2 an dem Implantationsort sichert. Zur weiteren Erhöhung der Befestigungssicherheit kann eine Zackung 28 an dem Führungsstab 21 ausgebildet sein, die im eingesetzten Zustand der Prothese 2 in das den Schlitz 18 umgebende Knochenmaterial einschneidet. Weiter kann eine Lasche 4 zur zusätzlichen Befestigung mittels einer Schraube (nicht dargestellt) vorgesehen sein. Weiter ist es zweckmäßig, wenn die Druckplatte 24 und der Führungsstab 21 zumindest an ihren zur Anlage an dem Wirbel 1 bestimmten Flächen mit einer wachstumsfördernden Beschichtung, wie Hydroxylapatit, beschichtet sind.

In Fig. 3 ist weiter ein nicht zur Erfindung gehörendes Ausführungsbeispiel dargestellt, bei welchem an dem dem Lagerstück 23 gegenüberliegenden Ende des Führungsstabs 21 eine kugelartige Verdickung 29 angeordnet ist. Sie ist aufgenommen in einer Erweiterung 19, die an dem Schlitz 18 in einer der Länge des Führungsstabs 21 entsprechenden Distanz von der Gelenkfläche ausgebildet ist. Diese Erweiterung kann durch den Chirurgen bei der Implantation leicht mittels eines Spiralbohrers von der Seite her erzeugt werden. Dank der rotationssymmetrischen Wölbung der kugelartigen Verdickung 29 an ihrer zu dem Lagerstück 23 gewandten Seite kann so eine nahezu flächigbündige Anlage erreicht werden, und zwar auch dann, wenn der Führungsstab 21 zur Fixierung 90 Grad verdreht wird, wie vorstehend beschrieben.

Die Lagerschale 27 kann eine ebene Außenseite als Gelenkfläche aufweisen. Bevorzugt ist es aber, ihr eine konvexe (für die superiore Gelenkfläche 13) oder eine konkave Gestaltung (für die inferiore Gelenkfläche 14) zu geben; eine umgekehrte Gestaltung ist aber ebenfalls möglich. Die Kontur des Lagerstücks 23 ist zweckmäßigerweise so gewählt, dass die zum Ersatz der inferioren Lagerfläche 14 vorgesehene Lagerschale 27 eine rechteckförmige Umhüllende aufweist (beispielsweise die Gestalt eines Rechtecks mit abgerundeten Kanten, siehe Fig. 1). Demgegenüber weist bei einer Therapie der superioren Lagerfläche 13 die Lagerschale vorzugsweise eine kreisartige Kontur auf (siehe Fig. 5 und nachstehende Erläuterung).

Eine Ausführungsform der Erfindung ist in Fig. 5 und 6 dargestellt. Gleiche Teile sind mit der gleichen Bezugsziffer versehen. Diese Ausführungsform der erfindungsgemäßen Prothese 3 unterscheidet sich von der in Fig. 1 und 2 dargestellten illustrativen Ausführungsform 2 im wesentlichen dadurch, dass ein zweites Lagerstück 33 am gegenüber liegenden Ende des Führungsstabs 21 vorgesehen ist. Der Führungsstab 21 ist zweiteilig ausgeführt mit einem zusätzlichen inneren Führungsstab 31. Durch Einschieben des inneren Führungsstabs 31 kann der Abstand des oberen Lagerstücks 23 von dem unteren Lagerstück 33 verändert werden. Vorzugsweise sind Verrastungsmittel 39 als Sicherung vorgesehen. Es sei angemerkt, dass auch alternative Ausführungen zur Längenveränderung möglich sind, beispielsweise mittels eines Schraubgewindes 30 (s. Fig. 5b). Das untere Lagerstück 33 ist ebenfalls über ein Schwenkgelenk mit dem Führungsstab 31 winkelbeweglich verbunden. Zweckmäßigerweise ist es als ein in zwei rotatorischen Freiheitsgraden bewegliches Kardangelenk 32 ausgeführt.

Zur Vereinfachung des Einschraubens bei der in Fig. 5b dargestellten Ausführungsform ist vorzugsweise an einer Außenseite des Lagerstücks 33, nämlich der Lagerschale 37, eine Aufnahme für ein Drehwerkzeug ausgebildet. In der dargestellten Ausführungsform ist eine sechseckige Vertiefung 38 vorgesehen. Sie ist vorzugsweise so angeordnet, dass sie in axialer Verlängerung des inneren Führungsstabs 31 liegt. Damit kann auf einfache Weise mittels eines Sechskantschraubenschlüssels, gegebenenfalls nach Verschwenken des darüber befindlichen Wirbels 1 ", der innere Führungsstab 31 in den Führungsstab 21 eingeschraubt werden. Bei ungünstigen Zugangsbedingungen kann dies auch noch bei einem leicht schräg stehenden unteren Lagerstück 33 durchgeführt werden; größere Winkel sind hierbei möglich, wenn das Schwenkgelenk als ein Kardangelenk 32 ausgebildet ist. Damit ist es ermöglicht, über die Lagerstücke 23, 33 Druck auf den Wirbel 1 auszuüben. Damit kann Knochenwachstum in dem Wirbel 1' angeregt werden, so dass die Prothese 3 schnell und sicher in den Wirbel 1' einwächst.

In Fig. 6 ist die Ausführungsform der erfindungsgemäßen Prothese 3 im implantierten Zustand gezeigt. Man erkennt, dass das Lagerstück 33 auf der oberen Gelenkfläche 13 und das Lagerstück 23 auf der unteren Gelenkfläche 14 des Wirbels 1' aufliegt. Die Prothese 3 eignet sich damit zur Therapie der Facettengelenke 12 in zwei übereinander liegenden Etagen. In den benachbarten Wirbeln können mit einem Lagerstück 23 versehene Prothese 2, wiederum sich über zwei Etagen erstreckende Prothesen 3 oder ggf. auch gar keine Prothese angeordnet sein. Dank der an dem physiologischen Vorbild orientierten Gestaltung des Lagerstücks 23, 33 sowie deren Lagerschalen 27, 37 ist es ermöglicht, dass die Prothese 2, 3 mit ihrer Lagerschale 37 mit der natürlichen Gelenkfläche 13, 14 des benachbarten Wirbels 1 zusammenwirkt. Die Lagerschale 27 wirkt wie in Fig. 3 dargestellt mit der Prothese 2 in dem Wirbel 1 zusammen.

Zur Implantation der Ausführungsform der erfindungsgemäßen Prothese 3 ist entsprechend wie bei der illustrativen Ausführungsform vorzugehen. Der Schlitz 18' ist aber über die gesamte Höhe des Gelenkfortsatzes 15 ausgebildet.

Nachfolgend wird eine Operationstechnik für die erfindungsgemäße Prothese erläutert. Danach wird ein posteriorer Zugang zur Wirbelsäule durchgeführt. Nach dem Freilegen erfolgt ein Öffnen einer das zu therapierende Facettengelenk 12 umgebenden Gelenkkapsel (nicht dargestellt). Da für die Implantation der erfindungsgemäßen Prothese 2, 3 nur ein verhältnismäßig klein bemessener Zugang erforderlich ist, genügt ein Öffnen der Gelenkkapsel in der Form, dass sie nur minimal geschädigt wird. Sie kann im übrigen verbleiben; ein Entfernen der Gelenkkapsel ist anders als bei herkömmlichen Operationsverfahren nicht erforderlich. Im Gegenteil, nach erfolgter Implantation der Prothese kann die Kapsel wieder hergestellt werden. Der Zugang zu dem Facettengelenk 12 ist nach dem Öffnen der Gelenkkapsel frei. Es wird dann in einem nächsten Schritt von dorso-lateral mittels eines an sich bekannten Turbinenfräsers ein Schlitz 18, 18' an dem Gelenkfortsatz der Wirbel 1, 1' ausgebildet, in die die erfindungsgemäße Prothese 2, 3 zu implantieren ist. Der Schlitz 18, 18' erstreckt sich dabei in der Richtung von der oberen Gelenkfläche 13 zu der unteren Gelenkfläche 14 des jeweiligen Gelenkfortsatzes 15. Die Länge des Schlitzes 18, 18' kann wie erforderlich gewählt werden, beispielsweise zur Implantation der illustrativen Ausführungsform der Prothese 2 auf einen Bereich nahe dem betroffenen Facettengelenk 12 und zur Implantation der erfindungsgemäßen Ausführungsform 3 über die gesamte Höhe des Gelenkfortsatzes 15. Wie bereits erwährt, kann die Weite des Schlitzes 18, 18' klein gewählt sein, um ein schnelles Zuwachsen zu begünstigen. Die Prothese 2, 3 wird dann mit ihrem Führungsstab 21, 31 in den Schlitz 18, 18' eingebracht und so weit eingeschoben, bis die Lagerstücke 23, 33 die vorgesehene Position auf den Gelenkflächen 13, 14 der zu therapierenden Facettengelenke 12 erreicht haben. Gegebenenfalls erfolgt dann eine Fixierung der Prothese 2, 3 durch Verdrehen des ovalförmig oder rechteckig gestalteten Schafts 21. Soweit erforderlich wird bei der Prothese gemäß der erfindungsgemäßen Ausführungsform 3 die Länge des Führungsstabs 21, 31 so weit variiert, bis beide Lagerstücke 23, 33 mit ihren jeweiligen Druckplatten 24, 34 fest auf dem Wirbeln aufliegen. Die Implantation der Prothese 2, 3 ist damit beendet. Nach Verschließen der Operationswunde ist eine Initialstabilisierung der Prothese 2, 3 durch eine Verdrehung des ovalen Schafts 21 erreicht, und langfristig ist dank der geringen erforderlichen Weite des Schlitzes 18, 18' mit einem Zuwachsen zu rechnen. Damit erfolgt eine dauerhafte und biokompatible Fixierung der Prothese.

## Patentansprüche

1. Prothese für Facettengelenke der Wirbelsäule mit einem Lagerstück (23) und einem Haltestück mit einem Führungsstab (21), wobei das Lagerstück (23, 33) eine Druckplatte (24) zur Auflage auf dem Wirbel (1) und gegenüber liegend eine Lagerschale (27, 37) umfasst, die eine Gelenkfläche zum Zusammenwirken mit einer Gelenkfläche eines benachbarten Wirbels (1') aufweist, **dadurch gekennzeichnet, dass**
zur Befestigung des Führungsstabs (21) an dem Lagerstück (23) ein Schwenkgelenk (22) vorgesehen ist und an dem anderen Ende des Führungsstabs (21) ein zweites Lagerstück (33) mit Druckplatte (34) und Lagereinsatz (37) angeordnet ist.

2. Prothese nach Anspruch 1,
**dadurch gekennzeichnet, dass**
das Schwenkgelenk ein Kardangelenk (32) ist.

3. Prothese nach Anspruch 1 oder 2,
**dadurch gekennzeichnet, dass**
ein teleskopartig in den Führungsstab (21) greifender innerer Führungsstab (31) vorgesehen ist.

4. Prothese nach Anspruch 3,
**dadurch gekennzeichnet, dass**
Verriegelungsmittel (39) zum Sichern des Führungsstabs (21, 31) gegenüber einem Auseinanderbewegen vorgesehen sind.

5. Prothese nach einem der Ansprüche 1 bis 4,
**dadurch gekennzeichnet, dass**
das Lagerstück (23) am superioren Ende des Führungsstabs (21, 31) eine Kontur einer ovalartigen Umhüllenden und das Lagerstück (33) am inferioren Ende eine Kontur mit einer rechteckigen Umhüllenden aufweist.

6. Prothese nach einem der vorangehenden Ansprüche,
**dadurch gekennzeichnet, dass**
mindestens eines der Lagerstücke (23, 33) einen Schrauböffnung (38) in seiner Gelenkfläche aufweist und drehfest mit dem inneren Führungsstab (31) verbunden ist.

7. Prothese nach einem der vorangehenden Ansprüche,
**dadurch gekennzeichnet, dass**
das Lagerstück (23) mit Druckplatte (24) und Lagereinsatz (27) einstückig ausgebildet ist.

8. Prothese nach einem der vorangehenden Ansprüche,
**dadurch gekennzeichnet, dass**
der Führungsstab (21) zum Einbringen in einen Schlitz (18) zwischen Gelenkflächen (13, 14) eines Wirbels (1) ausgebildet ist.

9. Prothese nach einem der vorangehenden Ansprüche,
**dadurch gekennzeichnet, dass** der Führungsstab (21) einen unrunden, vorzugsweise rechteckigen oder ovalen Querschnitt (21) aufweist, dessen größte Weite größer ist als die Weite des Schlitzes (18).

10. Prothese nach einem der vorangehenden Ansprüche,
**dadurch gekennzeichnet, dass**
entlang des Führungsstabs (21) eine radial abstehende Zackung (28) vorgesehen ist.

11. Prothese nach einem der vorangehenden Ansprüche,
**dadurch gekennzeichnet, dass**
an der Druckplatte (24) eine in den Wirbel (1) greifende Verzahnung (25) vorgesehen ist.

12. Prothese nach einem der vorangehenden Ansprüche,
**dadurch gekennzeichnet, dass**
die Druckplatte (24, 34) mit einer Knochenwachstum fördernden Beschichtung versehen ist, insbesondere aus Hydroxylapatit.

13. Prothese nach einem der vorangehenden Ansprüche,
**dadurch gekennzeichnet, dass**
die Lagerschale (27, 37) eine Gelenkfläche mit konvexer Wölbung aufweist.

14. Prothese nach Anspruch 13,
**dadurch gekennzeichnet, dass**
die zusammenwirkende Gelenkfläche an dem benachbarten Wirbel (1') eine komplementäre Wölbung aufweist.

15. Prothesensatz umfassend mindestens eine Prothese nach einem der vorangehenden Ansprüche,
**dadurch gekennzeichnet, dass**
mindestens eine Prothese (3) mit zwei Lagerstücken (23, 33) und mindestens eine Prothese (2) mit einem Lagerstück (23) vorgesehenen sind.

## Claims

1. Prosthesis for facet joints of the spinal column, with a bearing piece (23) and a retaining piece with a guide rod (21), the bearing piece (23, 33) comprising a pressure plate (24) for resting on the vertebra (1) and, on the opposite side, a bearing shell (27, 37) which has an articular surface for interacting with an articular surface of an adjacent vertebra (1') **characterized in that** a pivot joint (22) is provided for securing the guide rod (21) on the bearing piece (23), and a second bearing piece (33) with pressure plate (34) and bearing insert (37) is arranged at the other end of the guide rod (21).

2. Prosthesis according to Claim 1, **characterized in that** the pivot joint is a cardan joint (32).

3. Prosthesis according to Claim 1 or 2, **characterized in that** an inner guide rod (31) is provided which engages in a telescopic manner in the guide rod (21).

4. Prosthesis according to Claim 3, **characterized in that** locking means (39) are provided for securing the guide rods (21, 31) against moving apart.

5. Prosthesis according to one of Claims 1 to 4, **characterized in that** the bearing piece (23) at the upper end of the guide rod (21, 31) has a contour with an oval envelope, and the bearing piece (33) at the lower end has a contour with a rectangular envelope.

6. Prosthesis according to one of the preceding claims, **characterized in that** at least one of the bearing pieces (23, 33) has a screw opening (38) in its articular surface and is connected in a rotationally fixed manner to the inner guide rod (31).

7. Prosthesis according to one of the preceding claims, **characterized in that** the bearing piece (23) with pressure plate (24) and bearing insert (27) is designed in one piece.

8. Prosthesis according to one of the preceding claims, **characterized in that** the guide rod (21) is designed for insertion into a slit (18) between articular surfaces (13, 14) of a vertebra (1).

9. Prosthesis according to one of the preceding claims, **characterized in that** the guide rod (21) has a non-circular, preferably rectangular or oval cross section (21) whose greatest width is greater than the width of the slit (18).

10. Prosthesis according to one of the preceding claims, **characterized in that** a radially protruding serration (28) is provided along the guide rod (21).

11. Prosthesis according to one of the preceding claims, **characterized in that** a toothing arrangement (25) engaging in the vertebra (1) is provided on the pressure plate (24).

12. Prosthesis according to one of the preceding claims, **characterized in that** the pressure plate (24, 34) is provided with a coating that promotes bone growth, in particular a coating of hydroxyapatite.

13. Prosthesis according to one of the preceding claims, **characterized in that** the bearing shell (27, 37) has an articular surface with a convex curvature.

14. Prosthesis according to Claim 13, **characterized in that** the interacting articular surface on the adjacent vertebra (1') has a complementary curvature.

15. Prosthesis set comprising at least one prosthesis according to one of the preceding claims, **characterized in that** at least one prosthesis (3) with two bearing pieces (23, 33) and at least one prosthesis (2) with one bearing piece (23) are provided.

## Revendications

1. Prothèse pour articulation à facettes de la colonne vertébrale avec une pièce d'appui (23) et une pièce de maintien avec une tige de guidage (21), dans laquelle la pièce d'appui (23, 33) comprend une plaque de pression (24) pour l'appui sur la vertèbre (1) et en opposition un coussinet d'appui (27, 37), qui présente une face d'articulation destinée à coopérer avec une face d'articulation d'une vertèbre voisine (1'), **caractérisée en ce qu'**il est prévu une articulation pivotante (22) pour la fixation de la tige de guidage (21) à la pièce d'appui (23) et une deuxième pièce d'appui (33) avec une plaque de pression (34) et un insert d'appui (37) est disposée à l'autre extrémité de la tige de guidage (21).

2. Prothèse selon la revendication 1, **caractérisée en ce que** l'articulation pivotante est une articulation à cardan (32).

3. Prothèse selon la revendication 1 ou 2, **caractérisée en ce qu'**il est prévu une tige de guidage interne (31) s'engageant de façon télescopique dans la tige de guidage (21).

4. Prothèse selon la revendication 3, **caractérisée en ce qu'**il est prévu des moyens de verrouillage (39) pour bloquer la tige de guidage (21, 31) contre un mouvement de désengagement.

5. Prothèse selon l'une quelconque des revendications 1 à 4, **caractérisée en ce que** la pièce d'appui (23) à l'extrémité supérieure de la tige de guidage (21, 31) présente un contour d'enveloppe ovale et la pièce d'appui (33) à l'extrémité inférieure présente un contour avec une enveloppe rectangulaire.

6. Prothèse selon l'une quelconque des revendications précédentes, **caractérisée en ce qu'**au moins une des pièces d'appui (23, 33) présente une ouverture de vis (38) dans sa face d'articulation et est assemblée de façon solidaire en rotation à la tige de guidage interne (31).

7. Prothèse selon l'une quelconque des revendications précédentes, **caractérisée en ce que** la pièce d'appui (23) est formée d'une seule pièce avec la plaque de pression (24) et l'insert d'appui (27).

8. Prothèse selon l'une quelconque des revendications précédentes, **caractérisée en ce que** la tige de guidage (21) est réalisée en vue de son introduction dans une fente (18) entre des faces d'articulation (13, 14) d'une vertèbre (1).

9. Prothèse selon l'une quelconque des revendications précédentes, **caractérisée en ce que** la tige de guidage (21) présente une section transversale (21) non ronde, de préférence rectangulaire ou ovale, dont la plus grande largeur est plus grande que la largeur de la fente (18).

10. Prothèse selon l'une quelconque des revendications précédentes, **caractérisée en ce qu'**il est prévu une denture (28) radialement saillante le long de la tige de guidage (21).

11. Prothèse selon l'une quelconque des revendications précédentes, **caractérisée en ce qu'**il est prévu sur la plaque de pression (24) une denture (25) pénétrant dans la vertèbre (1).

12. Prothèse selon l'une quelconque des revendications précédentes, **caractérisée en ce que** la plaque de pression (24, 34) est munie d'un revêtement favorisant la croissance osseuse, en particulier en hydroxyapatite.

13. Prothèse selon l'une quelconque des revendications précédentes, **caractérisée en ce que** le coussinet d'appui (27, 37) présente une face d'articulation avec une courbure convexe.

14. Prothèse selon la revendication 13, **caractérisée en ce que** la face d'articulation conjuguée sur la vertèbre voisine (1') présente une courbure complémentaire.

15. Ensemble de prothèses comprenant au moins une prothèse selon l'une quelconque des revendications précédentes, **caractérisé en ce qu'**il est prévu au moins une prothèse (3) avec deux pièces d'appui (23, 33) et au moins une prothèse (2) avec une pièce d'appui (23).
